# EUROPEAN PATENT SPECIFICATION

(11) **EP 4 021 209 B1**
(45) Date of publication and mention of the grant of the patent: **08.04.2026**
(21) Application number: 20757910.3
(22) Date of filing: 21.08.2020
(51) Int. Cl.: A23L 33/155, A61K 9/00, A61K 31/4415, A61K 31/525, A61K 31/593, A61K 31/714, A61K 35/747, A61K 35/00, A61P 15/00, A61P 31/00, A23L 33/125, A23L 33/135, A23L 33/16, A61K 33/30, A61K 35/745, A23L 33/15, A61K 31/047

(54) **MYO-INOSITOL AND THE PREVENTION OF PRETERM-ONSET PRE-LABOUR RUPTURE OF MEMBRANES (PPROM)**
MYO-INOSITOL UND DIE PRÄVENTION VON FRÜHZEITIGEM BLASENSPRUNG VOR DEN WEHEN (PPROM)
MYO-INOSITOL ET LA PRÉVENTION DES RUPTURES PRÉMATURÉES DES MEMBRANES PRÉTERME (RPMAT)

(30) Priority: 26.08.2019 EP 19193541; 22.06.2020 EP 20181446
(43) Date of publication of application: 06.07.2022
(73) Proprietor: Société des Produits Nestlé S.A., 1800 Vevey (CH)
(72) Inventor: CHAN, Shiao-Yng, Singapore 119228 (SG); GODFREY, Keith Malcolm, Hampshire SO40 7AP (GB); CUTFIELD, Wayne, 1142 AUCKLAND (NZ); SILVA ZOLEZZI, Irma, Singapore 276750 (SG)
(74) Representative: Gagliardi, Tatiana
(86) International application number: PCT/EP2020/073450
(87) International publication number: WO 2021/037698

(56) References cited:
- WO-A1-03/053447
- WO-A1-2016/020486
- WO-A1-2016/020491
- US-B2- 8 404 690
- VITAGLIANO AMERIGO ET AL: "Inositol for the prevention of gestational diabetes: a systematic review and meta-analysis of randomized controlled trials", ARCHIVES OF GYNECOLOGY AND OBSTETRICS, SPRINGER VERLAG, BERLIN, DE, vol. 299, no. 1, 18 December 2018 (2018-12-18), pages 55 - 68, XP036861776, ISSN: 0932-0067, [retrieved on 20181218], DOI: 10.1007/S00404-018-5005-0
- KEITH M. GODFREY ET AL: "Nutritional Intervention Preconception and During Pregnancy to Maintain Healthy Glucose Metabolism and Offspring Health ("NiPPeR"): study protocol for a randomised controlled trial", TRIALS, vol. 18, no. 1, 20 March 2017 (2017-03-20), XP055664302, DOI: 10.1186/s13063-017-1875-x
- PLOWS JASMINE F ET AL: "Nutritional Supplementation for the Prevention and/or Treatment of Gestational Diabetes Mellitus", CURRENT DIABETES REPORTS, CURRENT SCIENCE, PHILADELPHIA, VA, US, vol. 19, no. 9, 1 August 2019 (2019-08-01), pages 1 - 15, XP036874945, ISSN: 1534-4827, [retrieved on 20190801], DOI: 10.1007/S11892-019-1199-1

## Description

The present invention relates generally to the field of compositions comprising myo-inositol for use in the prevention of a preterm-onset pre-labour rupture of membranes (PPROM) and disorders and/or conditions linked thereto. The composition may be a nutritional composition, for example a maternal nutrition composition. The composition may be specifically designed to provide optimized nutrition to a woman desiring to get pregnant or to a pregnant woman.

PPROM is a pregnancy complication. In this condition, the amniotic membrane surrounding the baby ruptures prematurely. In humans, PPROM occurs before the 37^{th} week of pregnancy. A PPROM can lead to an increased risk for infections. There is also a higher chance that the baby is born preterm before the 37^{th} week of pregnancy.

PPROM happens in about 30% of preterm births. Prognosis is primarily determined by complications related to prematurity such as necrotizing enterocolitis, intraventricular hemorrhage, and cerebral palsy, as well as intrauterine infection.

Women who have had PPROM are usually at a higher risk of experiencing it again in future pregnancies. To the inventor's best knowledge, there is currently no recommended way to specifically prevent PPROM. Some have suggested that women with a history of preterm delivery, including those associated with PPROM, take progesterone supplementation to prevent a recurrence.

Generally, it is recommended that women take good care of themselves during pregnancy to prevent complications. Taking good care should include ensuring an optimal nutrition. In general, scientific evidence is accumulating that prenatal early nutrition causes programming of long-term health and well-being, and can impact the risk of developing chronic diseases. Several studies have shown that changes in dietary intake or manipulation of individual macro and micronutrients during the reproductive period can have an impact in several physiological processes, such as growth, metabolism, appetite, cardiovascular function among others (Koletzko B et al (2011) Am J Nutr 94(s):2036-43S).

Therefore, nutritional status (nutrient stores and dietary intake) of women before and during pregnancy is of relevance to optimize neonatal and child health outcomes. Maternal nutrition is thought to affect the availability and supply of nutrients to the developing fetus that are required for critical developmental processes. Inadequate intakes of multiple micronutrients are common among women of reproductive age living in resource poor-settings (Torhem LE et al. (2010) J. Nutr. 140: 2051S-58S).

A large variety of nutrients have already been used in compositions for maternal administration and various nutritional compositions have been developed to address maternal nutrition needs. These typically contain vitamin and mineral mixes.

WO2016020486A1 discloses a combination of zinc and myo-inositol for use to improve insulin sensitivity and/or to treat or prevent an impaired glucose tolerance and/or type II diabetes and/or gestational diabetes mellitus and/or to prevent a condition associated with any of the foregoing in a subject.

Keith M. GODFREY et al. (2017) Trials 18:131, 2-12) assessed in the double blind randomised controlled NiPPeR (Nutritional Intervention Preconception and During Pregnancy to Maintain Healthy Glucose Metabolism and Offspring Health) study the effect of supplementing women during pregnancy twice-daily with a nutritional drink enriched with additional micronutrients, myo-inositol and probiotics compared to twice-daily control nutritional drink, enriched with standard micronutrients.

WO2016020491A1 discloses myo-inositol and probiotics for use to improve the body composition of an infant, in particular to prevent excess weight and/or to increase the % lean mass of an infant, wherein said myo-54 inositol and probiotics are administered simultaneously, separately or sequentially to said infant in-utero and optionally also after birth.

However, it would still be useful to specifically target the nutritional deficiencies of this specific population by selecting the most useful nutrients for these women such as to provide compositions tailored to the nutritional needs of women in the reproductive period to optimize the prenatal nutrition of mothers, for the benefit of the mother and of her infant.

In the absence of a recommended intervention specifically to contribute to the prevention of a preterm-onset pre-labour rupture of membranes (PPROM), it would be desirable to have such an intervention available.

Further, a combination of optimal nutrition during pregnancy with a composition that is able to contribute to the prevention of preterm-onset pre-labour rupture of membranes (PPROM) would be desirable as well.

Any reference to prior art documents in this specification is not to be considered an admission that such prior art is widely known or forms part of the common general knowledge in the field.

Accordingly, there is a need to overcome one or more of the drawbacks of the prior art and/or to find a composition that can be used in the prevention of a preterm-onset pre-labour rupture of membranes (PPROM) and disorders and/or conditions linked to PPROM in a female subject.

It would therefore be desirable to provide a composition for use in the prevention of a preterm-onset pre-labour rupture of membranes (PPROM) and disorders and/or conditions linked to PPROM in a female subject; or at least to provide a useful alternative.

The objective of the present invention was it, hence, to enrich or improve the state of the art and in particular, to provide a compound and/or a composition for use in the prevention of a preterm-onset pre-labour rupture of membranes (PPROM) and disorders and/or conditions linked to PPROM in a female subject.

The inventors were surprised to see that the object of the present invention could be achieved by the subject matter of the independent claims. The dependent claims further develop the idea of the present invention.

Accordingly, the present invention provides a composition comprising myo-inositol for use in the prevention of a preterm-onset pre-labour rupture of membranes (PPROM) and disorders and/or conditions linked to PPROM in a female subject.

As used in this specification, the words "comprises", "comprising", and similar words, are not to be interpreted in an exclusive or exhaustive sense. In other words, they are intended to mean "including, but not limited to".

The present inventors were surprised to find that by administering a composition comprising myo-inositol to pregnant women, the risk of PPROM could be significantly reduced, leading, for example, to a reduction of preterm delivery associated with PPROM. To the best knowledge of the inventors, this represents the first nutritional intervention that has been found to reduce the occurrence of PPROM. Without wishing to be bound by theory, the inventors currently believe that this effect is achieved through anti-inflammatory, metabolic and microbiome-related actions.

The present invention, hence, as defined in the claims relates to a composition comprising myo-inositol for use in the prevention of a preterm-onset pre-labour rupture of membranes (PPROM) and disorders and/or conditions linked to PPROM in a female subject. Also disclosed herein is the use of a composition comprising myo-inositol in the prevention of a preterm-onset pre-labour rupture of membranes (PPROM) and disorders and/or conditions linked to PPROM in a female subject. The present disclosure further relates to a kit of parts comprising at least two physically separated compositions, each comprising at least one ingredient selected from the group consisting of from vitamin B2, vitamin B6, vitamin B12, and vitamin D, at least one of the physically separated compositions comprises myo-inositol and at least one of the physically separated compositions comprises probiotics, for use in the prevention of a of preterm-onset pre-labour rupture of membranes (PPROM) and disorders and/or conditions linked to PPROM in a female subject.
Figure 1 shows the nutritional composition used on Example 1, 2 and 3.
Figure 2 2 and Figure 2bis show the effect of the nutritional composition intervention on both Pre-labour Rupture of Membranes (PROM) and on Preterm-onset Pre-labour Rupture of Membranes (PPROM).
Figure 3 shows an effect of the nutritional composition on Preterm-Onset Pre-labour Rupture of Membranes (PPROM) in preterm deliveries.

Consequently, the present invention relates to a composition as defined in the claims comprising myo-inositol for use in the prevention of a preterm-onset pre-labour rupture of membranes (PPROM) and disorders and/or conditions linked to PPROM in a female subject.

The term "prevent" or "prevention" as used herein refers to the prevention of the occurrence, or reduction of the risk of the occurrence, of an unwanted condition, disorder or conditions associated with such disorders in a female subject.

The term "subject" as used herein refers to a mammal and more particularly a cat, a dog or a human. The human may be a woman, for example, a woman who is trying to get pregnant, or who is pregnant. In an embodiment of the invention the subject is a mammal selected from the group consisting of a cat, a dog and, a human. For example, the subject may a woman who is trying to get pregnant, or who is pregnant.

Myo-Inositol, or cis-1,2,3,5-trans-4,6-cyclohexanehexol, is the predominant isomeric form of inositol. Myo-Inositol is a compound present in animal and plant cells and plays an important role in various cellular processes, as the structural basis for secondary messengers in eukaryotic cells, in particular as inositol triphosphates (IP3), phosphatidylinositol phosphate lipids (PIP2/PIP3) and inositol glycans. Myo-inositol has been shown to participate in a variety of biological process such as cell growth and survival, development and function of peripheral nerves, osteogenesis, energy metabolism and reproduction (Croze et al. (2013) Biochimie 95:1811-1827). Myo-inositol is found as free form, phosphoinositides and phytic acid, in fresh fruits and vegetables, and in all foods containing seeds (beans, grains and nuts) (Clements RS and Darnell B. Am J Clin Nutr (1980) 33:1954-1967). Myo-Inositol from phytic acid can be released in the gut by phytases found in plants, microorganisms and in animal tissues (Schlemmer U et al. Mol Nutr Food Res (2009) 53:S330-S375). These enzymes are capable of releasing free inositol, orthophosphate, and intermediary products including the mono-, di-, tri-, tetra- and penta-phosphate forms of inositol. Much of the ingested inositol hexaphosphate is hydrolysed to inositol. Myo-inositol is also commercially available from several suppliers.

Disorders and/or conditions linked to PPROM are known to the skilled artisan. For example, the disorder and/or condition linked to a PPROM may be selected from the group consisting of infections, such as infections of the amniotic fluid and membranes, for example; the separation of the placenta from the uterus; complications with the umbilical cord; the necessity to deliver by surgical or cesarean section and combinations thereof.

Myo-Inositol is administered in accordance with the present invention in an effective amount. Typically, an effective amount will depend on the type, age, size, health status, lifestyle and/or genetic heritage of the subject. The effective amount may be split into several smaller amounts and administered throughout the day so as the total daily intake is the effective amount. A person skilled in the art will be able to propose appropriate amounts of myo-inositol to be consumed per day. The composition for use in accordance with the present invention provides myo-inositol in an amount of 0.2 to 5 g, preferably 1.5 to 5 g, more preferably 2 to 5 g, most preferably 2 to 4 g per daily dose.

The composition used in the framework of the present invention also comprises probiotics. It is particularly beneficial to combine myo-inositol with probiotics, as these have been found to improve the gut barrier function and to help nutrients pass through the gut (Cani PD et al. (2009) Gut 58:1091-1103). Combining myo-inositol with probiotics thus enhances the absorption of myo-inositol and other nutrients that may be present in the composition.

The term "probiotic" as used herein refers to live probiotic bacteria, non-replicating probiotic bacteria, dead probiotic bacteria, non-viable probiotic bacteria, fragments of probiotic bacteria such as DNA, metabolites of probiotic bacteria, cytoplasmic compounds of probiotic bacteria, cell wall materials of probiotic bacteria, culture supernatants of probiotic bacteria, and/or combinations of any of the foregoing. The probiotic may for example be live probiotic bacteria, non-replicating probiotic bacteria, dead probiotic bacteria, non-viable probiotic bacteria, or any combination thereof.

Preferably the probiotic is live probiotic bacteria.

More specially the composition used in the framework of the invention comprises from 10⁵ to 10¹² cfu (colony forming units) of Bifidobacterium lactis BB12 CNCMI-3446 and a from 10⁵ to 10¹² cfu of Lactobacillus rhamnosus GG CGMCC 1.3724, all amounts being defined by daily dose.

The composition used for the purpose of the present invention further comprises a combination of vitamins.

The present inventors were able to show (Crozier SR et al, Am J Clin Nutr. 2012;96:57-63; Godfrey KM et al, Diabetes. 2011;60:1528-34; Childs C et al., J Dev Orig Health Dis. 2015;6(Suppl 2):S36.; that pregnant women were more often deficient in vitamins B6, B12 and D compared to other nutrients. Also, they have demonstrated that vitamin B2 was not consumed in sufficient amounts by a significant proportion of the pregnant woman population. It is therefore of particular interest to supplement the diet of pregnant women with these vitamins in order to compensate these particularly often-occurring deficiencies.

The composition according to the invention provides from 0.14 to 14 mg of vitamin B2, from 0.19 to 19 mg of vitamin B6, from 0.26 to 26 µg of vitamin B12, from 1.5 to 100 µg of vitamin D, each per daily dose.

Most preferably, the composition for use in accordance with the present invention comprises 1.8 mg of vitamin B2, 2.6 mg of vitamin B6, 5.2 µg of vitamin B12 and 10 µg of vitamin D per daily dose.

Accordingly the composition for use in accordance with the present invention comprises from 0.2 to 5 g of myo-inositol, from 0.14 to 14 mg of vitamin B2, from 0.19 to 19 mg of vitamin B6, from 0.26 to 26 µg of vitamin B12, from 1.5 to 100 µg of vitamin D, from 10⁵ to 10¹² cfu of Bifidobacterium lactis BB12 CNCMI-3446 and a from 10⁵ to 10¹² cfu of Lactobacillus rhamnosus GG CGMCC 1.3724, all amounts being defined by daily dose.

The inventors have also provided evidence (Patent No.14719) of common zinc deficiencies in at least certain populations of pregnant woman, even though in a lesser extent than vitamins B2, B12, B6 and D. It is therefore also particularly advantageous to supplement the diet of pregnant women with zinc. Thus, the composition for use in accordance with the present invention may comprise zinc. Zinc may be present in an amount of from 1.1 to 40 mg per daily dose.

Additional vitamins and minerals may also be added. For example, vitamins and minerals may be added in accordance with the recommendations of Government bodies such as the USRDA. For example, the composition may contain one or more of the following micronutrients, calcium, magnesium, phosphorus, iron, zinc, copper, iodine, selenium, vitamin A or retinol activity equivalent (RAE) for example in the form of beta carotene or a mix of carotenoids, Vitamin C, Vitamin B1, niacin, folic acid, biotin, Vitamin E. Preferably the composition may contain one or more of the following micronutrients in the following amounts: 100 to 2500 mg calcium, 35 to 350 mg magnesium, 70 to 3500 mg phosphorus, 2.7 to 45 mg iron, 1.1 to 40 mg zinc, 0.1 to 10 mg copper, 22 to 1,100 µg iodine, 6 to 400 µg selenium, 77 to 3000 µg of vitamin A or retinol activity equivalent (RAE) for example in the form of beta carotene or a mix of carotenoids, 8.5 to 850 mg Vitamin C, 0.14 to 14 mg Vitamin B1, 1.8 to 35 mg niacin, 60 to 1000 µg folic acid, 3 to 300 µg biotin, 1.9 to 109 µg Vitamin E.

The composition for use in accordance with the present invention may advantageously further comprise at least one oil selected from long chain polyunsaturated fatty acids, such as arachidonic acid (ARA), eicosapentaenoic acid (EPA) and/or docosahexaenoic acid (DHA), in any suitable amount as known by the person skilled in the art, for example in an amount of 100-500 mg per daily dose, more preferably between 200 and 400 mg per daily dose.

Any of the nutrients mentioned herein may be used in any amount that is effective in achieving the objective of the present invention. Skilled artisans will be able to determine appropriate dosages. Typically, dosage will depend on age, size and health status of the mother, on her lifestyle, as well as on her genetic heritage.

Typically, amounts are defined in the present application as amounts per daily dose. The amount of nutrient may thus be selected in each composition accordingly depending upon whether it is intended to be consumed once a day or more or less frequently.

The nutrients may be provided as a sustained release formulation. This way, the nutrient can be consumed less frequently, while the body is still constantly supplied with sufficient amount of such nutrient.

The composition of the present invention may be intended for maternal administration. This means that the composition may to be administered to a female desiring to get pregnant, or to a pregnant female. The composition may be to be administered to a woman desiring to get pregnant, or to a pregnant woman. Preferably, the composition for use in accordance with the present invention is to be administered to a woman desiring to get pregnant and/or to a pregnant woman, most preferably to a pregnant woman.

The composition for use in accordance with the present invention may be to be administered to a woman desiring to get pregnant, for example during at least 1, 2, 3 or 4 months preceding the pregnancy or desired pregnancy. When the composition is to be administered to a pregnant woman, the composition is preferably administered for at least 4, preferably at least 8, more preferably at least 12, more preferably at least 16, more preferably at least 20, more preferably at least 24, more preferably at least 28, even more preferably at least 36 weeks during pregnancy. As the nutritional requirements increase in the second and third trimester of pregnancy, it is further preferred to administer the composition for use in accordance with the present invention throughout the third trimester of pregnancy and most preferably throughout the second and third trimesters of pregnancy.

It is important that women desiring to get pregnant prepare their body to the pregnancy by taking appropriate nutrients. Then, women's nutrients needs increase during pregnancy. The composition for use in accordance with the present invention is specifically designed to meet the needs of the woman during this period.

In an embodiment of the present invention, the composition for use in accordance with the present invention may be to be administered to a subject at risk of premature delivery and/or PPROM.

Subjects at risk of PPROM are well known to the skilled artisan. For example, women are at risk of PPROM if they have a previous history of PPROM, are smokers, or have a cervical cerclage.

Subjects at risk of premature delivery are also well known to the person skilled in the art and include subjects that have had a premature baby in the past, subjects that are pregnant with multiples, subjects with abnormalities in the uterus or cervix, subjects that are underweight or overweight before pregnancy, and subjects with a family history of premature birth.

The composition can be in any form that is suitable to administer all the ingredients. For example, it can be in the form of a powdered nutritional composition to be reconstituted in milk or water, a food product, a drink, a nutritional supplement or a nutraceutical.

When the composition is in the form of a powdered nutritional composition to be reconstituted in milk or water it preferably comprises a protein source, a carbohydrate source and a lipid source, preferably together with lecithin. It may also comprise soya lecithin and/or a bulking agent. The protein source may be dried milk or dried skimmed milk. As carbohydrate source sucrose and/or maltodextrin may be used. The lipid source may be vegetable oil. The formulation may also alternatively or additionally contain glucose syrup, milk fat, magnesium citrate, choline salts and esters, prebiotic fibers, and/or ascorbyl palmitate. Flavor compounds, such as cocoa powder or honey, for example, may be added to provide taste variations.

The composition may be any type of composition suitable for consumption for the subject to whom it is to be administered.

The composition may also be a product selected from the group consisting of a nutritional product, a functional food product, a healthy ageing product, a dairy product, a dairy alternative product, a beverage product, a diet product, and a pet food product.

The term "nutritional product", as used herein, means any product that can be used to provide nutrition to a subject. Typically, nutritional products contain a protein source, a carbohydrate source and a lipid source.

The term "food product", as used herein, refers to any kind of product that may be safely consumed by a human or an animal. Said food product may be in solid, semisolid or liquid form and may comprise one or more nutrients, foods or nutritional supplements. For instance, the food product may additionally comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form).

The term "functional food product", as used herein, refers to a food product providing an additional health-promoting or disease-preventing function to the individual.

The term "healthy ageing product", as used herein, refers to a product providing an additional health-promoting or disease-preventing function related to healthy ageing to the individual.

The term "dairy products", as used herein, refers to food products produced from milk or fractions of milk from animals such as cows, goats, sheep, yaks, horses, camels, and other mammals. Examples of dairy products are low fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term "dairy alternative product" refers to products similar to dairy products but produced without milk.

The term "milk" is defined by Codex Alimentarius as the normal mammary secretion of milking animals obtained from one or more milkings without either addition to it or extraction from it, intended for consumption as liquid milk or for further processing.

The term "beverage product" as used herein, refers to a nutritional product in liquid or semi-liquid form that may be safely consumed by an individual.

The term "diet product" as used herein, refers to a food product with a restricted and/or reduced caloric content.

The term "pet food product" as used herein refers to a nutritional product that is intended for consumption by pets. A pet, or companion animal, as referenced herein, is to be understood as an animal selected from dogs, cats, birds, fish, rodents such as mice, rats
The term "nutritional supplement" as used herein, refers to a nutritional product that provides nutrients to an individual that may otherwise not be consumed in sufficient quantities by said individual. For instance, a nutritional supplement may include vitamins, minerals, fiber, fatty acids, or amino acids. Nutritional supplements may for example be provided in the form of a pill, a tablet, a lozenge, a chewy capsule or tablet, a tablet or capsule, or a powder supplement that can for example be dissolved in water or sprinkled on food. Nutritional supplements typically provide selected nutrients while not representing a significant portion of the overall nutritional needs of a subject. Typically, they do not represent more than 0.1%, 1%, 5%, 10% or 20% of the daily energy need of a subject. A nutritional supplement may be used during pregnancy, e.g., as a maternal supplement.

All ingredients of the composition can be admixed together or alternatively the composition can be provided in the form of a kit of parts wherein ingredients or groups of ingredients are provided separately. These separate compositions may be intended to be consumed separately or together.

Therefore a kit of parts for use in the prevention of a of preterm-onset pre-labour rupture of membranes (PPROM) and disorders and/or conditions linked to PPROM in a female subject comprising at least two physically separated compositions each comprising at least one of the ingredients mentioned above, wherein at least one of the physically separated compositions comprises myo-inositol and at least one of the physically separated compositions comprises probiotics, is also disclosed herein.

The kit of parts comprises at least two physically separated compositions, each comprising at least one ingredient selected from the group consisting of from vitamin B2, vitamin B6, vitamin B12, and vitamin D, at least one of the physically separated compositions comprises myo-inositol and at least one of the physically separated compositions comprises probiotics, for use in the prevention of a of preterm-onset pre-labour rupture of membranes (PPROM) and disorders and/or conditions linked to PPROM in a female subject.

Each of the physically separated compositions can further comprise one or more of the other ingredients mentioned above. Most preferably, a first composition comprises at least one vitamin selected from vitamin B2, vitamin B6, vitamin B12, vitamin D and mixtures thereof and a second composition comprises probiotics. Myo-inositol can be provided in the first or the second composition or even separately, but is preferably provided in the same composition as the vitamins. In a preferred embodiment of the invention, the kit of parts comprises a first composition comprising myo-inositol and at least one vitamin selected from vitamin B2, vitamin B6, vitamin B12, vitamin D and mixtures thereof, optionally with other vitamins and/or nutrients mentioned above, except probiotics, and a second composition comprising probiotics. More preferably, the kit of parts comprises a first composition comprising myo-inositol, vitamin B2, vitamin B6, vitamin B12, vitamin D and zinc and a second composition comprising probiotics. Separation of probiotics from other ingredients is mostly preferred to avoid any damage to the probiotics due to the presence of high concentration of minerals or other nutrients.

For the purpose of the present invention, the composition (or the compositions contained in the kit of part) is administered regularly, for example two times a day, daily, every two days or weekly.

Those skilled in the art will understand that they can freely combine all features of the present invention disclosed herein. Further, features described for different embodiments of the present invention may be combined.

Although the invention has been described by way of example, it should be appreciated that variations and modifications may be made without departing from the scope of the invention as defined in the claims.

The term "and/or" used in the context of the "X and/or Y" should be interpreted as "X", or "Y", or "X and Y".

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 1 to 8, from 3 to 7, from 4 to 9, from 3.6 to 4.6, from 3.5 to 9.9, and so forth.

All references to singular characteristics or limitations of the present invention shall include the corresponding plural characteristic or limitation, and vice versa, unless otherwise specified or clearly implied to the contrary by the context in which the reference is made.

Unless defined otherwise, all technical and scientific terms used herein have the same meaning as commonly understood by one of ordinary skill in the art.

Furthermore, where known equivalents exist to specific features, such equivalents are incorporated as if specifically referred in this specification. Further advantages and features of the present invention are apparent from the figures and non-limiting examples.

### Examples:

### Example 1

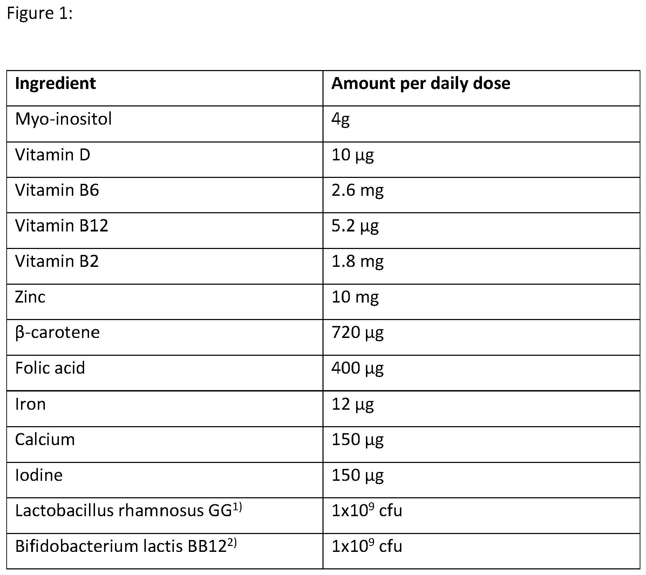

A nutritional supplement in powder form intended to be dissolved in water is provided with the ingredients as shown in figure 1.

Lactobacillus rhamnosus GG was deposited as CGMCC 1.3724.

Bifidobacterium lactis BB12 was deposited as CNCM I-3446.

The composition is provided as one sachet containing the probiotic and all other ingredients as a powder. The composition was administered to women desiring to get pregnant during at least one month prior to pregnancy and later to the same woman until delivery.

### Example 2

Five hundred eighty-five women participating in the NiPPeR trial (Godfrey KM et al; Trials (2017), 18: 131.) across three clinical sites were included in the analyses. This included 295 in the intervention and 290 in the control group. On average, women were 30 years of age, at the upper end of the normal weight range, and predominantly nulliparous. European and Chinese ethnicities accounted for >80% of ethnicities.

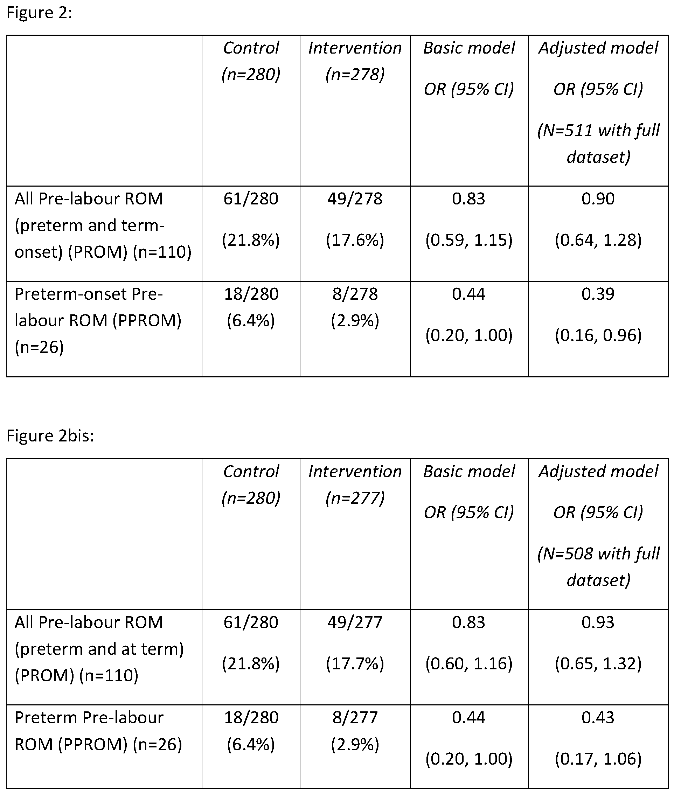

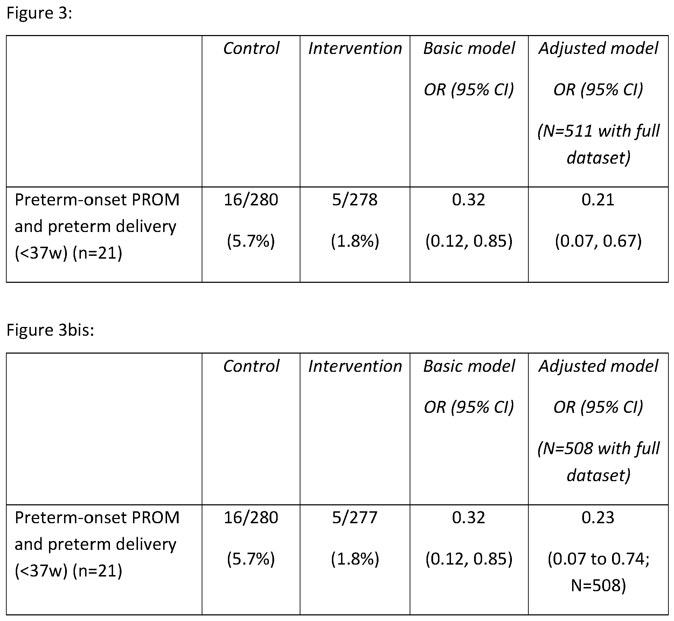

The nutritional intervention led to a statistically significant reduction in PPROM when adjustment was made for covariates including site and ethnicity ("basic model" in Figures 2 and 3), and additional for offspring's sex, maternal age, educational/ income level, parity, smoking during pregnancy, maternal pre-conception BMI, glycemia (preconception fasting glucose)("adjusted model" Figures 2 and 3).

A clear trend in the same direction was observed also when the same statistical analysis was carried out on a sample population whereby stillbirths were excluded and fasting glucose at pregnancy week 28 was used in the adjustment (Fig 2bis).

### Example 3

PPROM is a well recognised risk factor for preterm delivery. Subsequent analysis found a lower incidence of preterm delivery associated with PPROM in the intervention group than in the control group. The results are shown in figure 3.

Similar results were observed also when the same statistical analysis was carried out on a sample population whereby stillbirths were excluded and fasting glucose at pregnancy week 28 was used in the adjustment (Fig 3bis).

## Claims

1. Composition comprising myo-inositol for use in the prevention of preterm-onset pre-labour rupture of membranes (PPROM) and disorders and/or conditions linked to PPROM in a female subject, wherein the female subject is trying to get pregnant or is pregnant, and wherein the disorder and/or condition linked to a PPROM is selected from the group consisting of infections, such as infections of the amniotic fluid and membranes; the separation of the placenta from the uterus; complications with the umbilical cord; and combinations thereof;
wherein the composition comprises 0.2 to 5 g of myo-inositol, from 0.14 to 14 mg of vitamin B2, from 0.19 to 19 mg of vitamin B6, from 0.26 to 26 µg of vitamin B12, from 1.5 to 100 µg of vitamin D, from 10⁵ to 10¹² cfu of Bifidobacterium lactis BB12 CNCMI-3446 and a from 10⁵ to 10¹² cfu of Lactobacillus rhamnosus GG CGMCC 1.3724, all amounts being defined by daily dose

2. Composition for use in accordance with one of the preceding claims, wherein the composition further comprises zinc.

3. Composition for use in accordance with one of the preceding claims, wherein the subject is a mammal, for example selected from the group consisting of a cat, a dog and a human.

4. Composition for use in accordance with one of the preceding claims, wherein the composition is to be administered to a subject at risk of premature delivery and/or PPROM.

5. Composition for use in accordance with one of the preceding claims, wherein the composition is to be administered to a subject during pregnancy, for example during throughout the second and third trimesters of pregnancy.

6. Composition for use in accordance with one of the preceding claims, wherein the composition is in the form of a powdered nutritional composition to be reconstituted in milk or water, a food product, a drink, a nutritional supplement or a nutraceutical.

## Patentansprüche

1. Zusammensetzung, umfassend Myo-Inositol zur Verwendung bei der Prävention von vorzeitigem Blasensprung vor Wehenbeginn (PPROM) und Störungen und/oder Erkrankungen, die mit PPROM in Verbindung stehen, bei einem weiblichen Subjekt, wobei das weibliche Subjekt versucht schwanger zu werden oder schwanger ist, und wobei die Störung und/oder Erkrankung, die mit einem PPROM in Verbindung steht, ausgewählt ist aus der Gruppe, bestehend aus Infektionen, wie Infektionen des Fruchtwassers und Membranen; der Ablösung der Plazenta von der Gebärmutter; Komplikationen mit der Nabelschnur; und Kombinationen davon;
wobei die Zusammensetzung 0,2 bis 5 g Myo-Inositol, 0,14 bis 14 mg Vitamin B2, 0,19 bis 19 mg Vitamin B6, 0,26 bis 26 µg Vitamin B12, 1,5 bis 100 µg Vitamin D, von 10⁵ bis 10¹² KBE Bifidobacterium lactis BB12 CNCMI-3446 und von 10⁵ bis 10¹² KBE Lactobacillus rhamnosus GG CGMCC 1.3724 umfasst, wobei alle Mengen durch eine Tagesdosis definiert sind

2. Zusammensetzung zur Verwendung nach einem der vorstehenden Ansprüche, wobei die Zusammensetzung ferner Zink umfasst.

3. Zusammensetzung zur Verwendung nach einem der vorstehenden Ansprüche, wobei das Subjekt ein Säugetier ist, zum Beispiel ausgewählt aus der Gruppe, bestehend aus einer Katze, einem Hund und einem Menschen.

4. Zusammensetzung zur Verwendung nach einem der vorstehenden Ansprüche, wobei die Zusammensetzung einem Subjekt zu verabreichen ist, das ein Risiko für eine vorzeitige Entbindung und/oder PPROM aufweist.

5. Zusammensetzung zur Verwendung nach einem der vorstehenden Ansprüche, wobei die Zusammensetzung einem Subjekt während der Schwangerschaft zu verabreichen ist, zum Beispiel über das zweite und das dritte Trimester der Schwangerschaft hinweg.

6. Zusammensetzung zur Verwendung nach einem der vorstehenden Ansprüche, wobei die Zusammensetzung in der Form einer pulverförmigen Nährstoffzusammensetzung vorliegt, die in Milch oder Wasser, einem Nahrungsmittelprodukt, einem Getränk, einem Nahrungsergänzungsmittel oder einem Nutrazeutikum zu rekonstituieren ist.

## Revendications

1. Composition comprenant du myo-inositol pour utilisation dans la prévention de la rupture prématurée des membranes avant terme (RPMAT) et des troubles et/ou affections liés à la RPMAT chez un sujet féminin, dans laquelle le sujet féminin essaie de tomber enceinte ou est enceinte, et dans laquelle le trouble et/ou l'affection liés à une RPMAT est choisi dans le groupe constitué d'infections, telles que les infections du liquide amniotique et des membranes ; le décollement du placenta de l'utérus ; les complications liées au cordon ombilical ; et des combinaisons de ceux-ci ;
dans laquelle la composition comprend 0,2 à 5 g de myo-inositol, de 0,14 à 14 mg de vitamine B2, de 0,19 à 19 mg de vitamine B6, de 0,26 à 26 µg de vitamine B12, de 1,5 à 100 µg de vitamine D, de 10⁵ à 10¹² ufc de Bifidobacterium lactis BB12 CNCMI-3446 et de 10⁵ à 10¹² ufc de Lactobacillus rhamnosus GG CGMCC 1.3724, toutes les quantités étant définies par dose quotidienne

2. Composition pour utilisation selon l'une quelconque des revendications précédentes, dans laquelle la composition comprend en outre du zinc.

3. Composition pour utilisation selon l'une des revendications précédentes, dans laquelle le sujet est un mammifère, par exemple choisi dans le groupe constitué d'un chat, d'un chien et d'un humain.

4. Composition pour utilisation selon l'une des revendications précédentes, dans laquelle la composition doit être administrée à un sujet présentant un risque d'accouchement prématuré et/ou de RPMAT.

5. Composition pour utilisation selon l'une des revendications précédentes, dans laquelle la composition doit être administrée à un sujet au cours de la grossesse, par exemple tout au long des deuxième et troisième trimestres de la grossesse.

6. Composition pour utilisation selon l'une des revendications précédentes, dans laquelle la composition est sous la forme d'une composition nutritionnelle en poudre à reconstituer dans du lait ou de l'eau, d'un produit alimentaire, d'une boisson, d'un complément nutritionnel ou d'un produit nutraceutique.
